Europäisches Patentamt

European Patent Office

Office européen des brevets

(19)

(11) Numéro de publication : **0 097 175 B1**

(12) # FASCICULE DE BREVET EUROPÉEN

(45) Date de publication du fascicule du brevet :
28.05.86

(51) Int. Cl.⁴ : **B 65 D 81/32, H 02 G 1/14**

(21) Numéro de dépôt : **82903585.6**

(22) Date de dépôt : **08.12.82**

(86) Numéro de dépôt international :
**PCT/FR 82/00204**

(87) Numéro de publication internationale :
**WO/8302103 (23.06.83 Gazette 83/15)**

(54) CARTOUCHE POUR INJECTER UN PRODUIT SEMI-PATEUX.

(30) Priorité : 18.12.81 FR 8123670
24.09.82 FR 8216107

(43) Date de publication de la demande :
04.01.84 Bulletin 84/01

(45) Mention de la délivrance du brevet :
28.05.86 Bulletin 86/22

(84) Etats contractants désignés :
AT BE CH DE GB LI LU NL SE

(56) Documents cités :
FR-A- 749 096
FR-A- 1 599 814
FR-A- 2 178 733
FR-A- 2 357 311

(73) Titulaire : **ETABLISSEMENTS MOREL - ATELIERS ELECTROMECANIQUES DE FAVIERES (Société Anonyme)**
**Favières**
**F-28170 Chateauneuf-en-Thymerais (FR)**

(72) Inventeur : **MOREL, Jacques**
**Favières F-28170**
**Châteauneuf en Thymerais (FR)**
Inventeur : **MOREL, André**
**Favières F-28170**
**Châteauneuf en Thymerais (FR)**

(74) Mandataire : **Bouju, André**
**Cabinet Bouju 38 avenue de la Grande Armée**
**F-75017 Paris (FR)**

## Description

La présente invention concerne une cartouche pour injecter dans un corps creux, un produit semi-pâteux, tel qu'une mousse de polyuréthane obtenue par le mélange de deux constituants, à savoir un polyol et un isocyanate.

Par produit semi-pâteux on entend un produit dont la viscosité est comprise entre 500 et 2 000 centipoises.

La cartouche visée par l'invention est destinée en particulier à injecter de la mousse de polyuréthane dans un manchon d'étanchéité et d'isolation électrique entourant l'épissure qui raccorde des câbles électriques, téléphoniques ou électroniques.

On connaît selon le document FR-A-2 357 311 une cartouche pour injecter dans un corps creux, un produit semi-pâteux obtenu par le mélange de deux constituants, comprenant deux récipients, montés de façon coulissante l'un dans l'autre et renfermant chacun l'un des deux constituants précités. L'un des récipients est séparé de l'autre récipient par une cloison et celui-ci comporte un embout de sortie destiné à être raccordé à l'orifice d'injection du corps creux, cette cloison présentant un opercule perforable par une tige perforatrice montée axialement dans cette cartouche et cet embout présentant un opercule perforable pour permettre respectivement le passage des constituants du premier récipient dans le second récipient, et le passage du mélange résultant par l'embout de sortie du second récipient. Par ailleurs, la tige comporte une partie avant lisse et une partie filetée de diamètre supérieur à celui de la partie avant lisse, et le premier récipient comporte sur son extrémité opposée au second récipient une ouverture taraudée destinée à recevoir ladite partie filetée. La longueur de la partie avant lisse est supérieure à la distance comprise entre la cloison et l'ouverture taraudée du premier récipient.

L'expérience a montré que l'utilisation d'une telle cartouche présentait des difficultés dans le cas de produits présentant une viscosité supérieure ou égale à 1 000 centipoises.

En effet, l'injection de tels produits nécessite le déploiement d'efforts considérables pour faire coulisser le premier récipient dans le second récipient. De ce fait, on est obligé de limiter la dimension des cartouches, ce qui nécessite l'utilisation de plusieurs cartouches pour remplir des manchons de grand volume.

Le but de la présente invention est de remédier à ces inconvénients, en créant une cartouche facile à manipuler et pouvant contenir un volume de produit semi-pâteux notablement supérieur à celui des cartouches connues.

Suivant l'invention, cette cartouche est caractérisée en ce que la partie filetée est une partie intermédiaire comprise entre la partie avant et une partie arrière filetée, cette partie filetée intermédiaire ayant un diamètre supérieur aux diamètres desdites parties avant et arrière, en ce que l'extrémité libre de la partie avant lisse de la tige est filetée, en ce que l'embout de sortie du second récipient présente une ouverture taraudée destinée à recevoir le filetage ménagé sur l'extrémité de cette partie avant lisse, et en ce que la partie arrière filetée de la tige reçoit un écrou ayant un diamètre extérieur de même diamètre que celui de la partie filetée intermédiaire, destiné à prendre appui à l'extérieur de la cartouche, sur l'extrémité du premier récipient pour pousser ce dernier dans le second récipient.

Le fonctionnement de la cartouche conforme à l'invention est le suivant :

On engage axialement la tige dans l'ouverture taraudée du premier récipient. On visse la partie intermédiaire filetée de la tige dans cette ouverture. Lors de cette opération, l'extrémité de la tige perfore l'opercule ménagé sur la cloison de ce récipient. Le premier récipient communique ainsi avec le second récipient. On mélange les constituants contenus dans ce récipient en faisant coulisser plusieurs fois, dans les deux sens le premier récipient relativement au second récipient.

On enfonce ensuite davantage la tige dans la cartouche pour visser l'extrémité filetée de cette tige dans le taraudage pratiqué dans l'embout de sortie du second récipient. Dans cette position, la partie filetée de la tige fait saillie hors de l'ouverture taraudée du premier récipient. On visse l'écrou sur cette partie arrière filetée jusqu'à ce que celui-ci prenne appui sur l'extrémité adjacente du premier récipient, opposée à l'extrémité munie de la cloison. On continue ensuite à visser cet écrou, ce qui a pour effet de faire coulisser le premier récipient dans le second récipient, ce qui entraîne l'expulsion du produit résultant du mélange des deux constituants par l'embout de sortie du second récipient.

L'utilisation de la cartouche conforme à l'invention est par conséquent particulièrement commode et ne nécessite aucun effort particulier.

Une telle cartouche peut être de grande dimension, ce qui permet de remplir en une seule opération un corps creux de grand volume.

D'autres particularités et avantages de l'invention apparaîtront encore dans la description ci-après.

Aux dessins annexés, donnés à titre d'exemples non limitatifs :

la figure 1 est une vue en plan d'une cartouche conforme à l'invention,

la figure 2 est une vue en plan de la tige perforatrice de cette cartouche,

la figure 3 est une vue de l'écrou de commande du coulissement de l'un des récipients dans l'autre récipient de la cartouche,

la figure 4 est une vue de la cartouche en plan avec arrachements, montrant une phase préliminaire d'utilisation de la cartouche,

la figure 5 est une vue analogue à la figure 4 montrant la phase suivante d'utilisation de la cartouche,

les figures 6 et 7 sont des vues en plan de la cartouche, le premier récipient étant respectivement en position d'enfoncement et de dégagement,

la figure 8 est une vue de la cartouche en plan et avec arrachements, l'extrémité de la tige étant en position fixe dans l'embout de sortie du second récipient,

la figure 9 est une vue de l'extrémité de la cartouche montrant sa mise en place sur l'orifice d'injection d'un manchon de protection pour câble téléphonique,

la figure 10 est une vue en plan de la cartouche montrant la phase d'injection du produit dans le manchon, l'écrou de commande étant vissé sur la partie filetée de la tige,

la figure 11 est une vue partielle d'une autre réalisation de la tige,

la figure 12 est une vue en coupe suivant le plan XII-XII de la figure 11,

la figure 13 est une vue en coupe suivant le plan XIII-XIII de la figure 11,

la figure 14 est une vue en plan d'un levier de commande de la tige selon la figure 11,

la figure 15 est une vue en plan, avec arrachements d'une cartouche conforme à l'invention, montrant l'extrémité de la tige perforant la cloison du premier récipient,

la figure 16 est une vue analogue à la figure 15 montrant l'extrémité de la tige vissée dans le piston,

la figure 17 est une vue analogue à la figure 15 montrant le piston près de l'embout de sortie du second récipient et l'extrémité de la tige vissée dans cet embout,

la figure 18 est une vue en élévation et à échelle agrandie du piston,

la figure 19 est une vue en coupe suivant l'axe du piston,

la figure 20 est une vue en élévation éclatée du piston montrant ses deux éléments,

la figure 21 est une vue en plan montrant l'intérieur du piston,

la figure 22 est une vue en plan montrant l'intérieur de l'élément extérieur du piston,

les figures 23 et 24 sont des schémas illustrant le fonctionnement d'une cartouche conforme à l'invention.

Dans la réalisation des figures annexées, la cartouche pour injecter dans un corps creux un produit semi-pâteux tel qu'une mousse de polyuréthane obtenue par le mélange d'un polyol avec un isocyanate, comprend deux récipients cylindriques 1, 2, en matière plastique rigide, montés de façon coulissante l'un dans l'autre et renfermant chacun l'un des deux constituants précités.

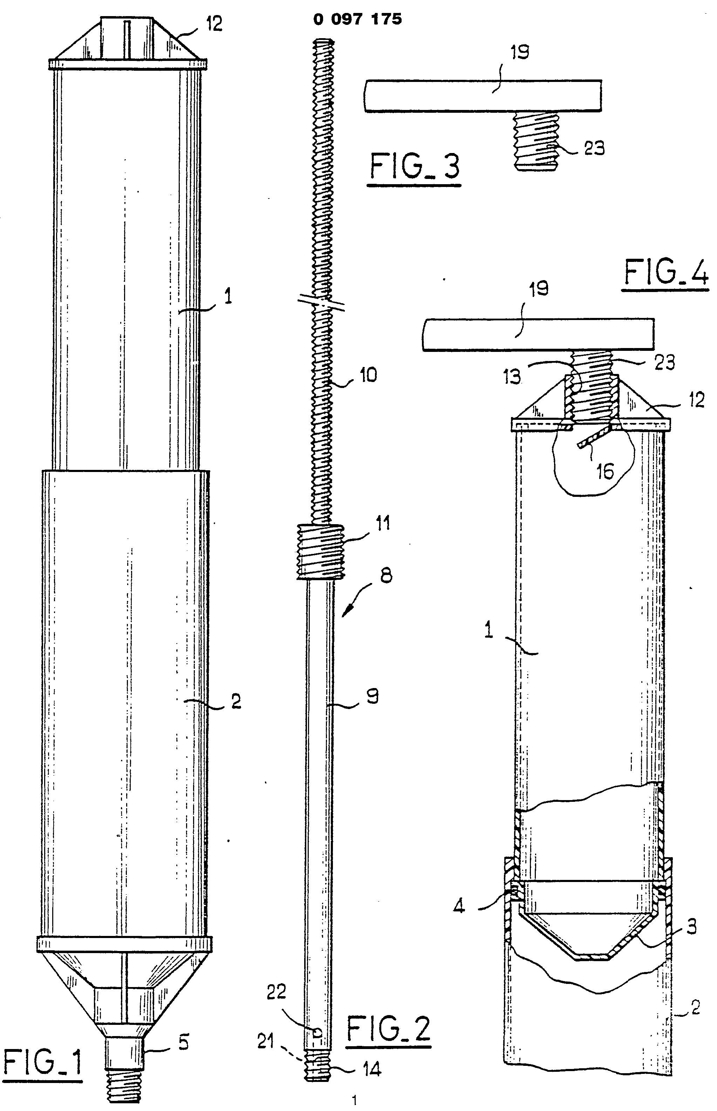

L'extrémité du récipient 1 engagée dans le récipient 2 est fermée par une cloison 3 sensiblement tronconique (voir figures 4, 5 et 8). Cette extrémité du récipient 1 présente un joint torique 4 assurant l'étanchéité entre les deux récipients 1, 2.

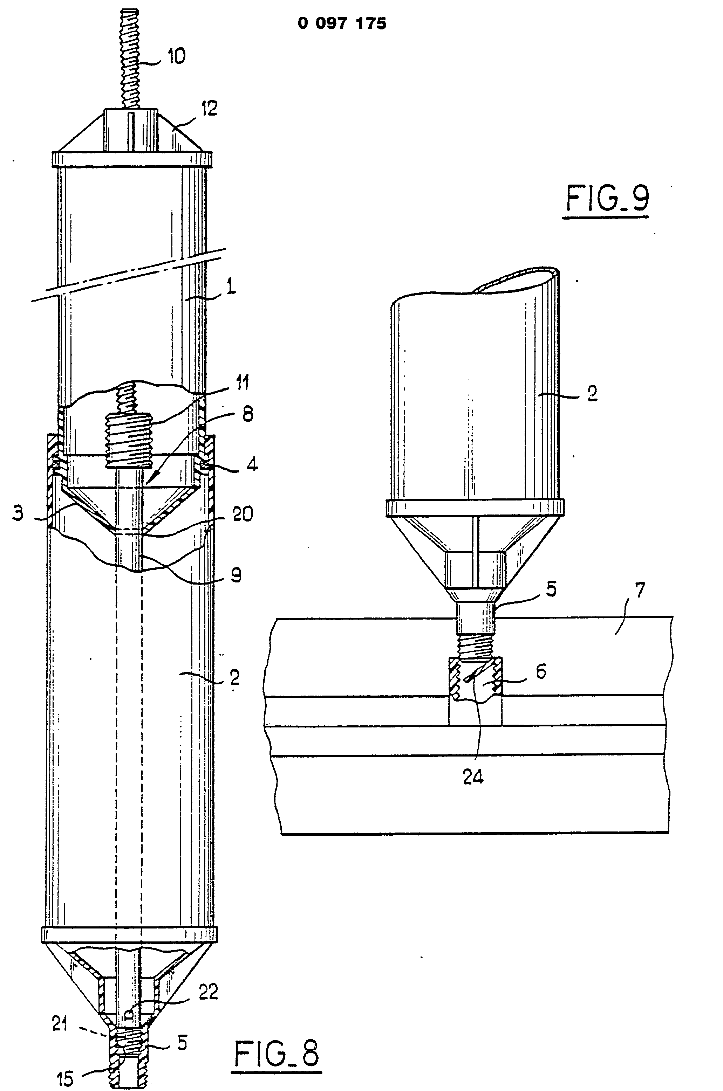

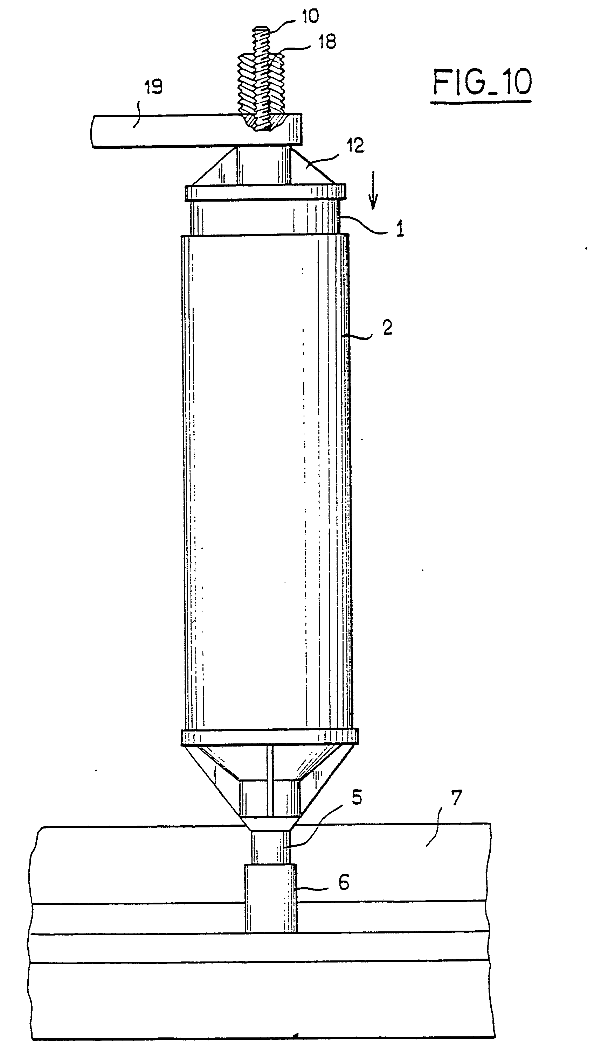

A l'opposé du récipient 1, le récipient 2 comporte un embout de sortie 5 (voir figures 1 et 6 à 10) destiné à être raccordé à l'orifice d'injection 6 d'un manchon 7 de protection pour câble téléphonique (voir figures 9 et 10).

La cartouche comprend d'autre part, une tige 8 (voir notamment figures 2 et 8) qui comporte une partie avant 9 cylindrique et lisse ainsi qu'une partie arrière filetée 10. Ces parties 9 et 10 sont séparées par une partie intermédiaire 11 courte et filetée, de diamètre supérieur à celui des parties avant et arrière 9, 10.

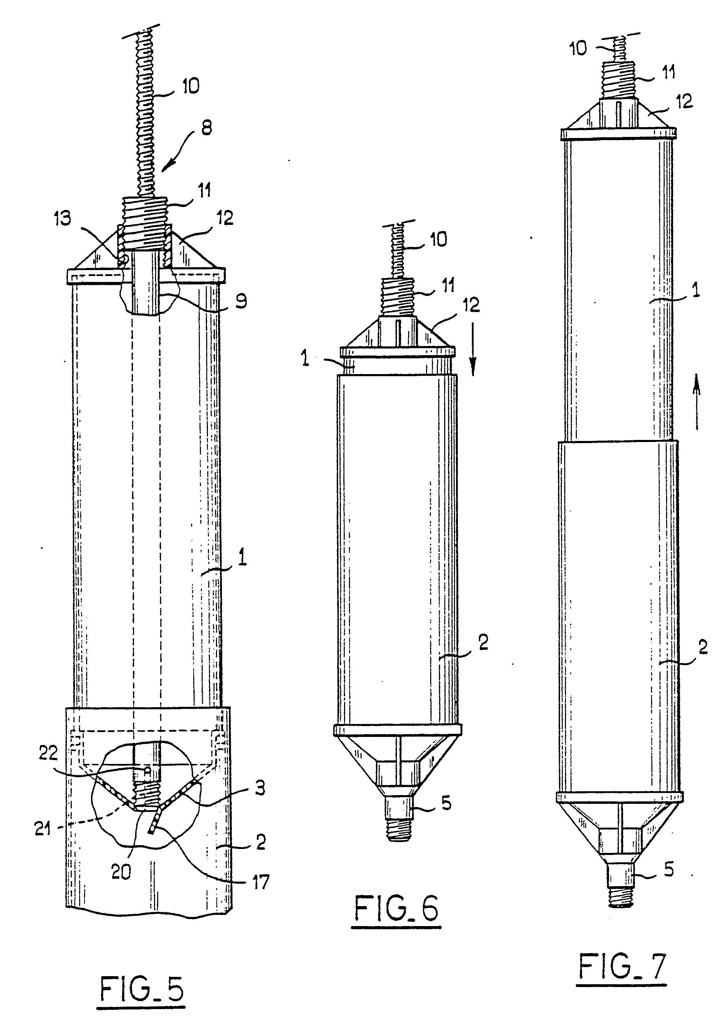

Le récipient 1 comporte sur son extrémité 12 opposée au récipient 2 une ouverture axiale taraudée 13, destinée à recevoir la partie intermédiaire filetée 11 de la tige 8, comme indiqué sur la figure 5.

L'extrémité libre de la partie lisse 9 de la tige 8 présente un filetage 14.

L'embout de sortie 5 du récipient 2 présente un taraudage interne 15 (voir figure 8) destiné à recevoir le filetage 14 ménagé sur l'extrémité de la tige 8.

La longueur de la partie lisse 9 de la tige 8 est légèrement supérieure à la distance comprise entre la cloison 3 du récipient 1 et le taraudage 13 ménagé sur l'extrémité 12 de ce récipient 1 (voir figure 5). Ce taraudage 13 comporte un opercule en matière plastique 16 destiné à être perforé ou enfoncé, comme indiqué sur la figure 4. De même, la cloison 3 du récipient 1 comporte un opercule perforable 17 (voir figure 5). Le taraudage 15 ménagé à l'intérieur de l'embout de sortie 5 du récipient 2 comporte également un opercule perforable qui n'est pas représenté sur les figures.

La partie filetée 10 de la tige 8 comporte un écrou 18 (voir figure 10) muni d'un levier latéral de commande 19. Dans la position représentée sur la figure 10, cet écrou 18 prend appui sur l'extrémité 12 du récipient 1.

Sur la figure 8, on voit que l'ouverture 20 définie après perforation de l'opercule 17 ménagé sur la cloison 3 du récipient 1, correspond à la section de la partie lisse 9 de la tige 8. L'étanchéité est ainsi réalisée entre cette partie lisse 9 et l'ouverture 20.

Par ailleurs, l'extrémité filetée 14 de la tige 8 présente un canal 21 (voir figures 2, 5 et 8) qui débouche latéralement sur la partie lisse 9 par un orifice 22 qui permet le passage du produit à injecter.

D'autre part, on voit notamment sur les figures 3, 4 et 10 que l'écrou 18 à levier 19 comporte un filetage extérieur 23 destiné à être vissé dans le taraudage 13 ménagé sur l'extrémité 12 du récipient 1 pour percer l'opercule 16 situé dans ce taraudage comme indiqué sur la figure 4.

On va maintenant expliquer le fonctionnement de la cartouche que l'on vient de décrire.

On supposera que le récipient 1 de la cartouche est rempli par un isocyanate et que le récipient 2 contient un polyol, ces deux constituants étant destinés à réagir ensemble pour former une mousse de polyuréthane.

Une opération préliminaire consiste à visser le filetage extérieur 23 de l'écrou à levier 19 dans le taraudage 13 ménagé sur l'extrémité 12 du récipient 1. Cette opération a pour effet d'enfoncer l'opercule 16 (voir figure 4) puis on dévisse le filetage extérieur (23) du taraudage (15).

On engage ensuite la partie lisse 9 de la tige 8 dans l'ouverture taraudée 13 et on visse la partie intermédiaire filetée 11 de cette tige 8 dans cette ouverture taraudée (voir figure 5).

L'extrémité filetée 14 touche l'opercule 17 ménagé sur la cloison 3, puis enfonce ce dernier. L'isocyanate s'écoule alors dans le récipient inférieur 2 en passant par le canal 21 ménagé sur l'extrémité 14 de la tige 8.

On accélère le mélange de l'isocyanate avec le polyol en enfonçant le récipient 1 dans le récipient 2 comme indiqué sur la figure 6 puis en le dégageant à nouveau comme indiqué sur la figure 7. On effectue ces deux mouvements plusieurs fois afin de réaliser un mélange homogène entre les deux constituants. Ces deux constituants réagissent ensemble pour former une mousse de polyuréthane ce qui se traduit par une importante augmentation de pression à l'intérieur du récipient 2.

On enfonce alors la tige 8 dans le récipient 2 et on visse son extrémité filetée 14 dans le taraudage 15 ménagé dans l'embout 5 (voir figure 8). La tige 8 est ainsi fixée solidement dans l'embout 5. On visse ensuite (voir figure 9) l'embout 5 dans l'orifice d'injection 6 ménagé sur le manchon 7, ce qui a pour effet d'enfoncer l'opercule 24 ménagé dans cet orifice 6.

On visse (voir figure 10) l'écrou 18 à levier 19 sur l'extrémité dépassante de la partie filetée 10 de la tige 8 jusqu'à ce que cet écrou 18 prenne appui sur l'extrémité 12 du récipient 1. En continuant à visser l'écrou 18 par rotation de son levier 19, le récipient 1 s'enfonce dans le récipient 2 et l'ouverture 20 ménagée sur la cloison 3 du récipient 1 coulisse de façon étanche sur la partie lisse 9 de la tige 8. La mousse de polyuréthane est ainsi expulsée progressivement et sans effort pour l'utilisateur, par le canal 21 ménagé dans l'extrémité filetée 14 de la tige 8.

On peut ainsi expulser de la cartouche des produits très visqueux (viscosité ≥ 1 000 centipoises) et en grande quantité. De ce fait, un manchon 7 de grand volume peut être rempli en une seule opération dans des conditions particulièrement commodes pour l'utilisateur.

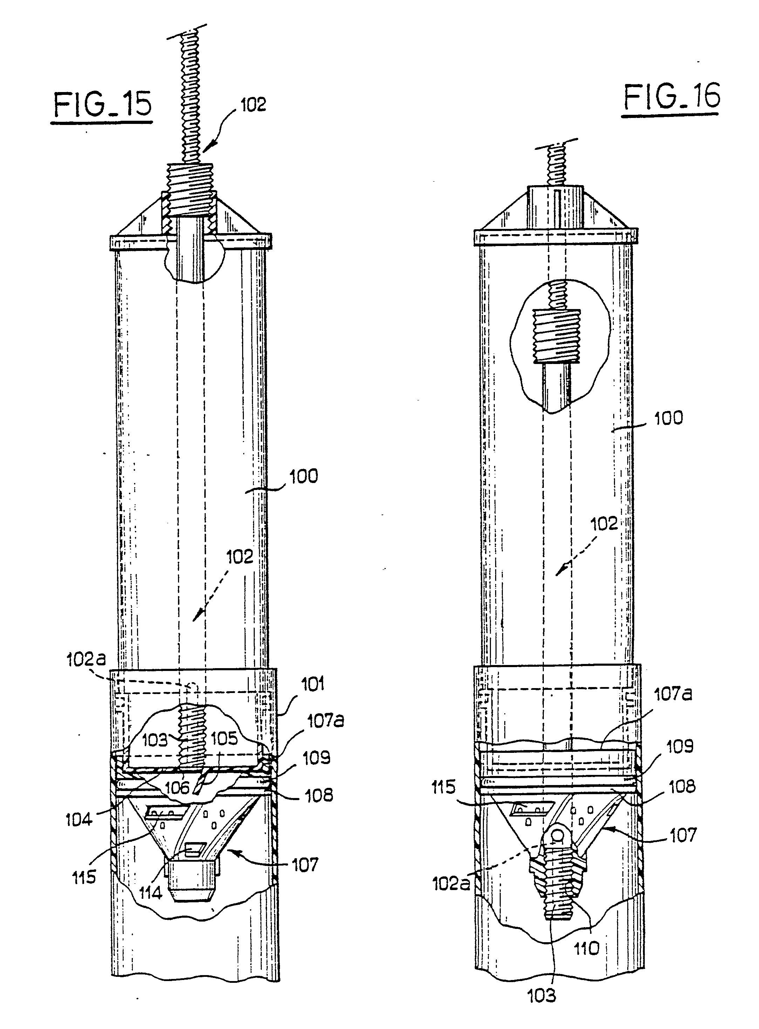

Dans la réalisation des figures 15 à 17, la cartouche comprend deux récipients cylindriques 100 et 101, montés coulissants l'un dans l'autre, et renfermant chacun un constituant du mélange à réaliser et à injecter.

Dans la position représentée sur la figure 15 une tige 102 est engagée axialement dans le premier récipient 100. L'extrémité filetée 103 de cette tige est en appui contre la cloison 104 du premier récipient 100 et perfore l'opercule 105 de cette cloison en ménageant ainsi dans cette dernière, un orifice 106.

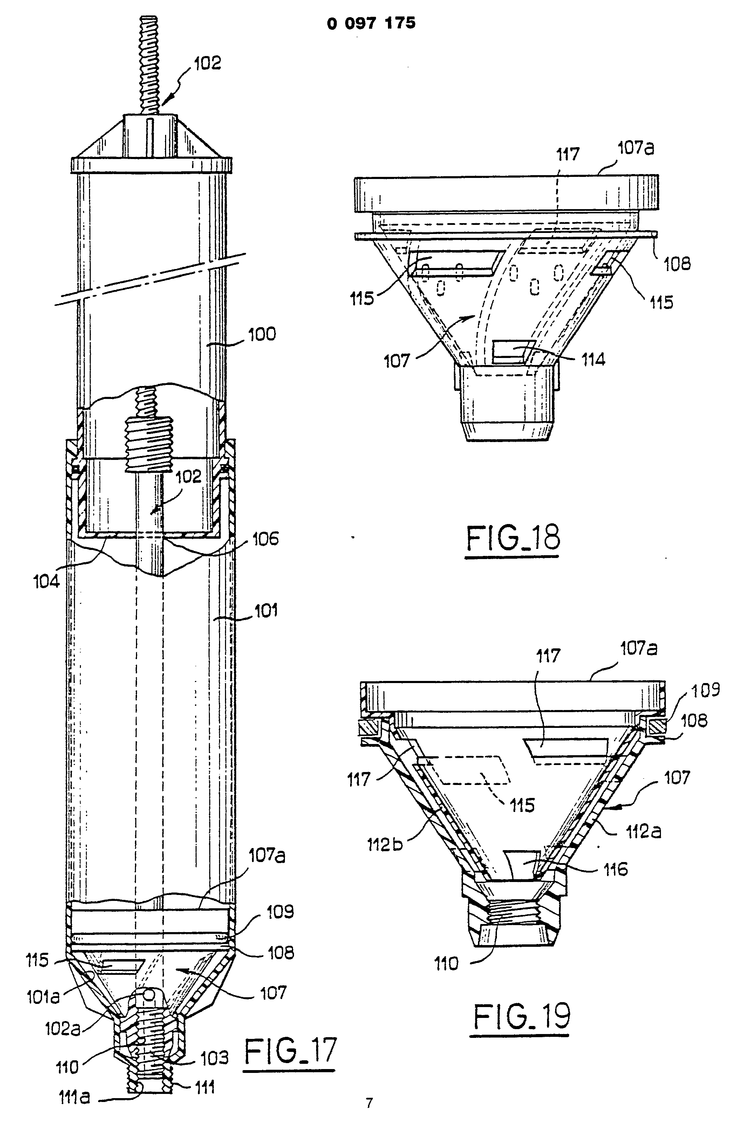

Cette cartouche comprend un piston 107 monté de façon coulissante dans le second récipient 101. Ce piston 107 est sensiblement tronconique. Sa surface extérieure tronconique est complémentaire de la surface interne tronconique 101a de l'extrémité du récipient 101, adjacente à son embout fileté 111 (voir figure 17).

Dans la position représentée sur la figure 15, la base 107/a du piston 107 est emboîtée autour de la cloison 104 et elle comporte une collerette 108 dans laquelle est inséré un segment 109 (voir figure 19) pour râcler la matière le long de la paroi du récipient 101.

A l'opposé de la base 107a, le piston 107 présente une ouverture axiale filetée 110 pouvant recevoir l'extrémité filetée 103 de la tige 102, comme indiqué sur la figure 16.

Cette extrémité filetée 103 de la tige 102 présente une longueur supérieure à la partie filetée de l'ouverture 110 du piston 107, pour pouvoir faire saillie hors de cette dernière et se visser dans le filetage interne 111a de l'embout de sortie 111 du second récipient 101 (voir figure 17).

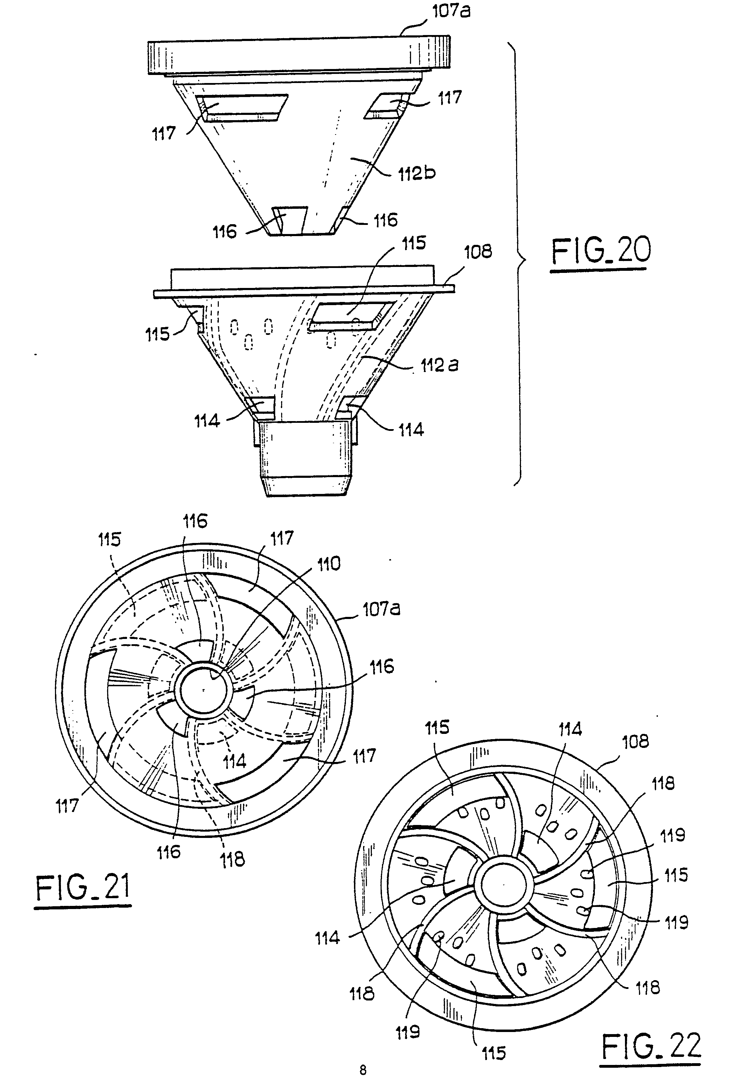

On voit notamment sur les figures 19 et 20 que le piston 107, comprend deux éléments tronconiques 112a et 112b assemblés axialement l'un dans l'autre, de façon à laisser entre eux un espace 113. Chaque élément 112a, 112b comprend deux séries d'ouvertures 114, 115, 116, 117.

Les ouvertures les plus grandes 115, 117 sont situées près de la base 107a du piston et les ouvertures les plus petites 114, 116 sont situées à l'opposé de cette base.

Dans la réalisation représentée, chaque série comprend trois ouvertures régulièrement réparties sur des cercles centrés sur l'axe du piston 107.

Les ouvertures 114 de l'élément extérieur 112a sont décalées angulairement par rapport aux ouvertures 115. De même, les ouvertures 116 de l'élément intérieur 112b sont décalées angulairement par rapport aux ouvertures 117.

De plus, les ouvertures 114, 115 de l'élément extérieur 112a, sont décalées angulairement par rapport aux ouvertures 116, 117 de l'élément intérieur 112b.

L'espace 113 compris entre les éléments de piston 112a, 112b est défini par des nervures curvilignes 118 qui s'étendent sur la face interne de l'élément extérieur 112a, entre l'orifice central fileté 110 et la collerette 108 (voir figures 21 et 22).

Ces nervures curvilignes 118 délimitent entre les ouvertures 114 de l'élément extérieur 112a et les ouvertures 117 de l'élément intérieur 112b, d'une part, et entre les ouvertures 116 de l'élément intérieur 112b et les ouvertures 115 de l'élément extérieur 112a d'autre part, des passages s'étendant suivant des trajectoires curvilignes dont la section croît entre l'extrémité libre du piston 107 et sa base 107a.

On voit également sur la figure 22 que la surface intérieure de l'élément extérieur 112a présente entre les nervures 118 des saillies 119.

Le fonctionnement de la cartouche que l'on vient de décrire est le suivant :

On engage la tige 102 dans le premier récipient 100 et on perfore l'opercule 105 de la cloison 104 (voir figure 15). On établit ainsi une communication entre les deux récipients 100 et 101 par le canal interne 102a de l'extrémité 103 de la tige.

On continue à enfoncer la tige 102 et on visse son extrémité filetée 103 dans l'ouverture filetée 110, ménagée à l'extrémité du piston 107 (voir figure 16).

On déplace alors le piston 107 vers l'extrémité du second récipient 101, puis on le ramène à nouveau en arrière, contre la cloison 104. On procède ainsi plusieurs fois de suite.

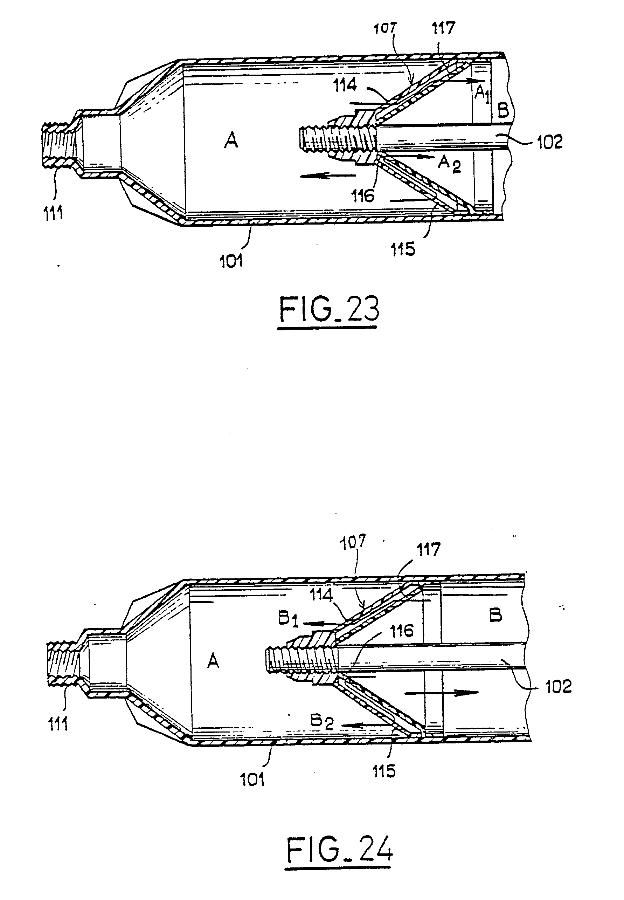

Lorsque le piston 107 est déplacé vers l'extrémité du second récipient 101, le constituant A (voir figure 23) contenu dans le récipient 101 entre dans le piston 107 par les ouvertures 114, passe dans les passages curvilignes compris entre les nervures 118 et sort de ces passages par les ouvertures 117 (voir flèche A₁ sur la figure 23). Le constituant A est ainsi prélevé près de l'axe du récipient 101, puis est mélangé avec le constituant B dans une zone située à la périphérie.

Lors de cette opération, le constituant A entre également dans le piston 107 par les ouvertures 115, et en sort par les ouvertures 116 (voir flèche A₂ sur la figure 23). Le constituant A est ainsi également prélevé à la périphérie, puis mélangé avec le constituant B, dans une zone située près de l'axe.

Lorsque le piston est déplacé en arrière (voir figure 24), c'est-à-dire vers le premier récipient 100, le constituant B contenu dans celui-ci entre dans le piston 107 par les ouvertures 117 et 116 et en sort par les ouvertures 114 et 115 (voir flèches B₁ et B₂).

Le constituant B est ainsi prélevé près de l'axe et de la périphérie du récipient 101 et mélangé avec le constituant A dans des zones situées respectivement près de la périphérie et près de l'axe du récipient.

Les saillies 119 situées dans les passages compris entre les ouvertures précitées permettent de diviser l'écoulement de la matière, ce qui permet d'améliorer l'homogénéité du mélange des constituants A et B.

Lorsque le mélange des constituants A et B est terminé, on visse l'extrémité filetée 103 de la tige 102 dans le filetage 111a de l'embout 111, ce qui a pour effet d'appliquer le piston 107 contre la surface interne tronconique 101a de l'extrémité du récipient 101.

On procède ensuite comme décrit plus haut pour injecter le mélange obtenu dans un corps creux tel qu'un manchon de protection pour câbles téléphoniques.

Bien entendu, la présente invention n'est pas limitée à l'exemple de réalisation que l'on vient de décrire, et on peut apporter à celui-ci de nombreuses modifications, sans sortir du cadre de l'invention.

Ainsi, des moyens d'étanchéité complémentaires peuvent être prévus au niveau de l'ouverture 20 de la cloison 3 du récipient 1.

L'extrémité 14 de la tige 8 pourrait être aménagée de façon à faciliter la perforation des différents opercules.

Bien entendu, la cartouche conforme à l'invention peut être utilisée pour injecter d'autres produits semi-pâteux qu'une mousse de polyuréthane et résultant du mélange d'au moins deux constituants liquides et/ou solides.

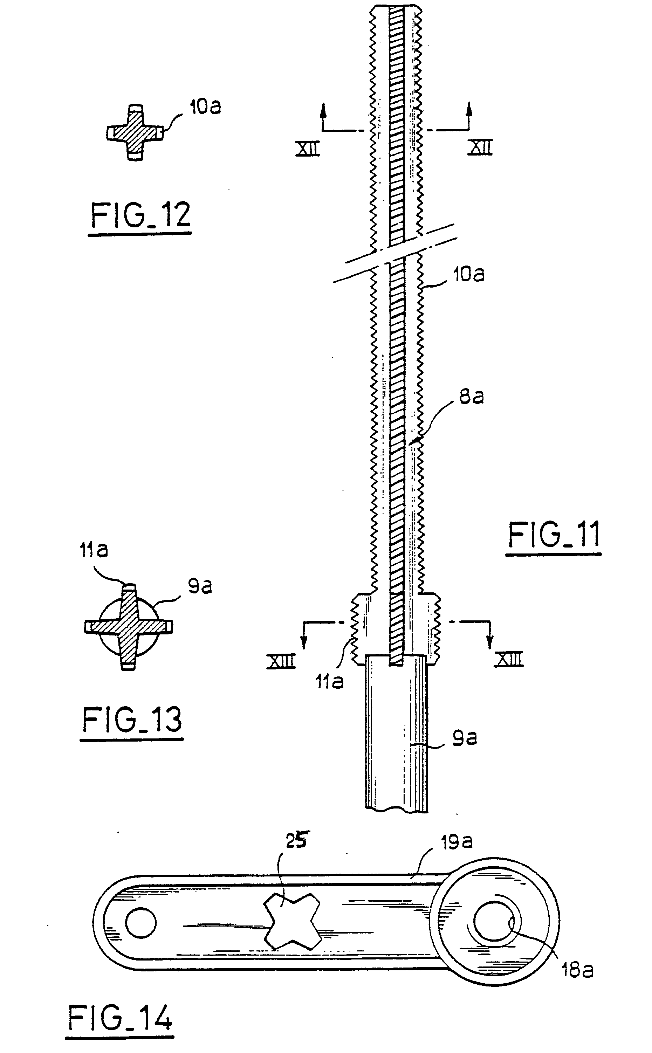

Dans la réalisation des figures 11 à 13, la partie arrière filetée 10a de la tige 8a comporte une section en étoile à quatre branches (voir figure 12). La partie avant lisse 9a est identique à la partie 9 de la tige 8 représentée sur la figure 2. La partie intermédiaire filetée 11a présente également une section en étoile (voir figure 13).

Le levier 19a représenté sur la figure 14 et qui porte l'écrou 18a destiné à être vissé sur la partie filetée 10a de la tige 8a comporte une ouverture 25 dont la section en étoile est adaptée à celle de la partie filetée 10a de la tige 8a.

Après introduction de la tige 8a dans la cartouche, on engage l'ouverture 25 du levier sur la partie filetée 10a de la tige 8a. Ce levier facilite considérablement la rotation de la tige 8a lors de la perforation de l'opercule 17.

Ainsi, l'embout de sortie 111 de la cartouche comporte un filetage extérieur permettant de visser cette cartouche sur un support fixe, ce qui faciliterait la manipulation de la cartouche lors de l'opération de mélange des constituants A et B, et permettrait d'injecter à distance le produit semi-pâteux dans un corps creux.

**Revendications**

1. Cartouche pour injecter dans un corps creux (7) un produit semi-pâteux obtenu par le mélange de deux constituants, comprenant deux récipients (1, 2) montés de façon coulissante l'un dans l'autre et renfermant chacun l'un des deux constituants précités, l'un (1) des récipients étant séparé de l'autre récipient (2) par une cloison (3), l'autre récipient (2) comportant un embout de sortie (5) destiné à être raccordé à l'orifice d'injection (6) du corps creux, cette cloison (3) présentant un opercule (17) perforable par une tige perforatrice (8) montée axialement dans cette cartouche et cet embout (5) présentant un opercule perforable pour permettre respectivement le passage des constituants du premier récipient (1) dans le second récipient (2), et le passage du mélange résultant par l'embout de sortie (5) du second récipient (2), la tige (8) comportant une partie avant lisse (9) et une partie filetée (11), de diamètre supérieur à celui de la partie avant lisse (9), le premier récipient (1) comportant sur son extrémité (12) opposée au second récipient (2) une ouverture taraudée (13) destinée à recevoir ladite partie filetée (11), la longueur de cette partie avant lisse (9) étant supérieure à la distance comprise entre la cloison (3) et l'ouverture taraudée (13) du premier récipient, caractérisée en ce que la partie filetée (11) est une partie intermédiaire comprise entre la partie avant (9) et une partie arrière filetée (10), cette partie filetée (11) ayant un diamètre supérieur aux diamètres desdites parties avant (9) et arrière (10), en ce que l'extrémité libre (14) de la partie avant lisse (9) de la tige est filetée, en ce que l'embout de sortie (5) du second récipient présente une ouverture taraudée (15) destinée à recevoir le filetage (14) ménagé sur l'extrémité de cette partie avant lisse (9), et en ce que la partie arrière filetée (10) de la tige (8) reçoit un écrou (18) ayant un diamètre extérieur (23) de même diamètre que celui de la partie filetée (11), destiné à prendre appui à l'extérieur de la cartouche, sur l'extrémité (12) du premier récipient (1) pour pousser ce dernier (1) dans le second récipient (2).

2. Cartouche conforme à la revendication 1, caractérisée en ce que l'ouverture (20) définie après perforation de l'opercule (17) ménagé sur la cloison (3) du premier récipient (1) a un diamètre sensiblement égal à celui de la partie lisse (9) de la tige (8).

3. Cartouche conforme à l'une quelconque des revendications 1 ou 2, caractérisée en ce que les ouvertures taraudées (13, 15) ménagées sur l'extrémité (12) du premier récipient (1) et dans l'embout de la sortie (5) du second récipient (2) comportent chacun un opercule (16) perforable au moyen de l'écrou (18).

4. Cartouche conforme à l'une quelconque des revendications 1 à 3, caractérisée en ce que l'extrémité filetée (14) de la partie avant lisse (9) de la tige (8) présente un canal (21) qui débouche latéralement sur la partie avant lisse (9) de la tige par un orifice (22) permettant le passage du produit à injecter.

5. Cartouche conforme à l'une quelconque des revendications 1 à 4, caractérisée en ce que l'écrou (18) comporte un levier (19) pour l'entraîner en rotation.

6. Cartouche conforme à la revendication 5, caractérisée en ce que la partie arrière filetée (10a) de la tige (8a) présente une section en étoile et en ce que le levier (19a) a écrou (18a) comporte une ouverture (25) adaptée à la section précitée.

7. Cartouche conforme à l'une quelconque des revendications précédentes, caractérisée en ce qu'elle comprend un piston (107) monté de façon coulissante dans le second récipient (101), ce piston comportant une ouverture axiale taraudée (110) pouvant recevoir l'extrémité filetée (103) de la tige perforatrice (102), après perforation de la cloison (104) du premier récipient (100) par ladite extrémité (103), en ce que cette extrémité filetée (103) de la tige (102) présente une longueur supérieure de la partie filetée de l'ouverture axiale taraudée (110) du piston, pour pouvoir faire saillie hors de cette ouverture (110) et se visser dans le taraudage interne (111a) de l'embout de sortie (111) du second récipient (101) et en ce que le piston présente une série d'ouvertures (114, 115, 116, 117) permettant le passage du constituant contenu dans chaque récipient vers l'autre récipient et inversement, ces ouvertures étant disposées de manière à dévier la trajectoire du passage des constituants de l'axe d'un récipient (100, 101) vers la périphérie de celui-ci ou inversement lorsque le piston (107) est déplacé.

8. Cartouche conforme à la revendication 7, caractérisée en ce que le piston (107) est sensiblement tronconique, sa base (107a) adjacente à la cloison du premier récipient (100) portant une collerette (108) dans laquelle est engagé un joint annulaire (109) pour établir son étanchéité avec le second récipient (101), en ce qu'il comprend deux éléments tronconiques (112a, 112b) assemblés axialement l'un dans l'autre de façon à laisser un espace (113) entre eux et en ce que chaque élément comporte deux séries d'ouvertures (114, 115 ; 116, 117), l'une de ces séries étant située près de la base (107a) du piston, et l'autre à l'opposé de cette dernière, les ouvertures de l'un des éléments (112a, 112b) étant décalées angulairement par rapport aux ouvertures de l'autre élément.

9. Cartouche conforme à la revendication 8, caractérisée en ce que les deux éléments (112a, 112b) du piston (107) sont séparés par des nervures (118) qui délimitent des trajectoires curvilignes de passage des constituants.

10. Cartouche conforme à la revendication 9, caractérisée en ce que lesdites trajectoires curvilignes s'étendent, d'une part entre les différentes ouvertures (114) ménagées sur l'élément extérieur (112a) à l'opposé de sa base (107a) et les différentes ouvertures (117) décalées par rapport aux précédentes, ménagées sur l'élément intérieur (112b) près de la base du piston, et d'autre part entre les différentes ouvertures (115) ménagées sur l'élément extérieur (112a) près de sa base (107a) et les différentes ouvertures (116) décalées des précédentes, ménagées sur l'élément intérieur (112b) à l'opposé de sa base.

11. Cartouche conforme à la revendication 10, caractérisée en ce que les ouvertures (115, 117) des éléments (112a, 112b) situées près de la base du piston (107) sont plus grandes que celles situées à l'opposé de cette base et sont décalées angulairement entre elles.

12. Cartouche conforme à la revendication 8, caractérisée en ce que dans chaque élément (112a, 112b), les trois ouvertures de chaque série sont régulièrement réparties sur un cercle distinct centré sur l'axe du piston (107).

13. Cartouche conforme à l'une quelconque des revendications 8 à 12, caractérisée en ce que la surface extérieure tronconique du piston (107) est complémentaire de la surface interne tronconique (101a) de l'extrémité du second récipient (101) adjacente à l'embout de sortie (111).

14. Cartouche conforme à l'une quelconque des revendications 9 à 13, caractérisée en ce que la surface interne de l'un des éléments de piston porte des saillies (119) entre les nervures (118) qui délimitent des trajectoires curvilignes de passage des constituants.

15. Cartouche conforme à la revendication 14, caractérisée en ce que les nervures (118) et les saillies (119) sont ménagées sur la face interne de l'élément externe (112a).

16. Cartouche conforme à l'une quelconque des revendications 7 à 15, caractérisée en ce que l'embout de sortie (111) de la cartouche comporte un filetage extérieur permettant de visser cet embout sur un support.

## Claims

1. Cartridge for injecting into a hollow body (7) a semi-pasty product obtained by mixing two constituents, comprising two containers (1, 2), one of which is slidably mounted in the other and each of which contains one of the two constituents aforesaid, one of the containers (1) being separated from the other container (2) by a partition wall (3), the other container (2) having a discharge nozzle (5) which is intended to be connected to the injection orifice (6) of the hollow body, this partition wall (3) being provided with a closure (17) pierceable by a perforating rod (8) mounted axially within this cartridge and this nozzle (5) being provided with a pierceable closure in order to permit respectively the passage of the constituents from the first container (1) into the second container (2) and the passage of the resulting mixture through the discharge nozzle (5) of the second container (2), the rod (8) being provided with a smooth front portion (9) and a threaded portion (11) having a larger diameter than that of the smooth front portion (9), the first container (1) being provided on its end portion (12) opposite to the second container (2) with an internally threaded opening (13) which is intended to receive said threaded portion (11), the length of this smooth front portion (9) being greater than the distance between the partition wall (3) and the internally threaded opening (13) of the fist container, characterized in that the threaded portion (11) is an intermediate portion located between the front portion (9) and a threaded rear portion (10), the diameter of this threaded portion (11) being greater than the diameters of said front portion (9) and rear portion (10), that the free end (14) of the smooth front portion (9) of the rod is threaded, that the discharge nozzle (5) of the second container has an internally threaded opening (15) which is intended to receive the threaded portion (14) formed on the end of this smooth front portion (9), and that the threaded rear portion (10) of the rod (8) receives a nut (18) having an external diameter (23), which is the same as that of the threaded portion (11), and intended to bear externally of the cartridge on the end portion (12) of the first container (1) in order to thrust this latter (1) into the second container (2).

2. Cartridge in accordance with claim 1, characterized in that the opening (20) defined after perforation of the closure (17) formed on the partition wall (3) of the first container (1) has a diameter which is substantially equal to that of the smooth portion (9) of the rod (8).

3. Cartridge in accordance with either of claims 1 or 2, characterized in that the internally threaded openings (13, 15) formed in the end portion (12) of the first container (1) and in the discharge nozzle (5) of the second container (2) each comprise a closure (16) which can be perforated by means of the nut (18).

4. Cartridge in accordance with any one of claims 1 to 3, characterized in that the threaded end (14) of the smooth front portion (9) of the rod (8) has a bore (21) which opens laterally on the smooth front portion (9) of the rod through an orifice (22) which permits the passage of the product to be injected.

5. Cartridge in accordance with any one of claims 1 to 4, characterized in that the nut (18) has a lever (19) for driving it in rotation.

6. Cartridge in accordance with claim 5, characterized in that the threaded rear portion (10a) of the rod (8a) has a star-shaped section and that the lever (19a) for the nut (18a) has an opening (25) adapted to the section aforesaid.

7. Cartridge in accordance with any one of the preceding claims, characterized in that it comprises a piston (107) slidably mounted in the second container (101), this piston being provided with an intenally threaded axial opening (110) which is capable of receiving the threaded end (103) of the perforating rod (102) after perforation of the partition wall (104) of the first container of the first container (100) by said end (103), that this threaded end (103) of the rod (102) is of greater length than the threaded portion of the internally threaded axial opening (110) of the piston so that it can project from this opening (110) and can be screwed into the internally threaded portion (111a) of the discharge nozzle (111) of the second container (101), and that the piston has a set of openings (114, 115, 116, 117) which permit the flow of the constituent contained ineach container towards the other container and vice versa, these openings being disposed in such a manner as to divert the flow path of the constituents from the axis of a container (100, 101) towards the periphery of this latter or vice versa when the piston (107) is displaced.

8. Cartridge in accordance with claim 7, characterized in that the piston (107) is substantially frusto-conical, its base (107a) adjacent to the partition wall of the first container (100) being provided with a flange (108) in which is engaged an annular seal (109) for establishing a leak-tight seal with the second container (101), that it has two frusto-conical members (112a, 112b), axially assembled one inside the other in such a manner as to leave a space (113) between them, and that each member has two sets of openings (114, 115 ; 116, 117), one of these sets being located near the base (107a) of the piston and the other set being located at the end remote from this latter, the openings of one of the members (112a, 112b) being angularly displaced with respect to the openings of the other member.

9. Cartridge in accordance with claim 8, characterized in that the two members (112a, 112b) of the piston (107) are separated by ribs (118) which define curvilinear paths for the flow of constituents.

10. Cartridge in accordance with claim 9, characterized in that said curvilinear paths extend on the one hand between the different openings (114) provided in the outer member (112a) at the end remote from its base (107a) and the different openings (117) which are displaced with respect to the aforementioned openings provided in the inner member (112b) near the base of the piston, and on the other hand between the different openings (115) provided in the outer member (112a) near its base (107a) and the different openings (116) which are displaced with respect to the aforementioned openings provided in the inner member (112b) at the end remote from its base.

11. Cartridge in accordance with claim 10, characterized in that the openings (115, 117) of the members (112a, 112b) located near the base of the piston (107) are larger than those located at the end remote from this base and are angularly displaced with respect to each other.

12. Cartridge in accordance with claim 8, characterized in that, in each member (112a, 112b), the three openings of each set are uniformly distributed over a separate circle centred on the axis of the piston (107).

13. Cartridge in accordance with any one of claims 8 to 12, characterized in that the external frustoconical surface of the piston (107) is complementary to the internal frusto-conical surface (101a) of the end of the second container (101) adjacent to the discharge nozzle (111).

14. Cartridge in accordance with any one of claims 9 to 13, characterized in that the internal surface of one of the piston members is provided with projecting portions (119) between the ribs (118) which define the curvilinear flow paths of the constituents.

15. Cartridge in accordance with claim 14, characterized in that the ribs (118) and the projecting portions (119) are formed on the internal face of the outer member (112a).

16. Cartridge in accordance with any one of claims 7 to 15, characterized in that the discharge nozzle (111) of the cartridge has an external thread which serves to screw this nozzle onto a support.

**Patentansprüche**

1. Kartusche zum Einspritzen eines halbpastösen Produktes in einen Hohlkörper (7), das durch Mischung zweier Bestandteile erhalten wird, die in zwei ineinander berschiebbar gelagerten Behältern (1, 2) enthalten sind, von denen jeder einen der Bestandteile enthält, wobei der eine (1) der Behälter von dem anderen Behälter (2) durch eine Trennwand (3) getrennt ist, der andere Behälter einen Austrittsstutzen (5) aufweist, der zur Verbindung mit einer Einspritzöffnung (6) des Hohlkörpers bestimmt ist, die Trennwand (3) einen mit Hilfe einer in der Kartusche axial angeordneten Perforierstange (8) perforierbaren Deckel (17) besitzt und das Rohrstück (5) einen perforierbaren Deckel aufweist, um den Durchtritt des Bestandteils des ersten Behälters in den zweiten Behälter (2) bzw. den Durchtritt des resultierenden Gemisches durch den Austrittsstutzen (5) des zweiten Behälters (2) zu ermöglichen und wobei die Stange (8) einen vorderen glatten Teil (9) und einen mit einem Außengewinde versehenen, einen größeren Durchmesser als der vordere glatte Teil (9) aufweisenden Teil (11) besitzt, der erste Behälter (1) an seinem vom zweiten Behälter (2) abgewandten Ende (12) eine mit einem Innengewinde versehene, zur Aufnahme des mit dem Außengewinde versehenen Teils (11) bestimmte Öffnung (13) aufweist, und die Länge dieses vorderen glatten Teils (9) größer ist als die Entfernung zwischen der Trennwand (3) und der Gewindeöffnung (13) des ersten Behälters, dadurch gekennzeichnet, daß der mit Außengewinde versehene Teil (11) ein Zwischenstück zwischen dem vorderen Teil (9) und einem hinteren, mit einem Außengewinde versehenen Teil (10) ist, wobei dieser mit Außengewinde versehene Teil (11) einen Durchmesser besitzt, der größer ist als die Durchmesser des genannten vorderen (9) und hinteren Teils (10), daß das freie Ende (14) des vorderen glatten Teils (9) der Stange mit einem Außengewinde versehen ist, daß der Austrittsstutzen (5) des zweiten Behälters eine Gewindeöffnung (15) zur Aufnahme des am Ende dieses vorderen glatten Teils (9) angeordneten Außengewindes aufweist, u. daß der hintere Gewindeteil (10) der Stange (8) eine Mutter (18) aufnimmt, die einen dem Durchmesser des mit Außengewinde versehen Teils (11) entsprechenden Außendurchmesser besitzt und zur Anlage an der Außenseite der Kartusche an dem Ende (12) ersten Behälters (12) dient und diesen in den zweiten Behälter drückt.

2. Kartusche nach Anspruch 1, dadurch gekennzeichnet, daß die nach dem Durchstoßen des Deckels (17) in der Trennwand (3) des ersten Behälters (1) gebildete Öffnung (20) einen durchmesser besitzt, der im wesentlichen jenem des glatten Teils (9) der Stange (8) entspricht.

3. Kartusche nach Anspruch 1 oder 2, dadurch gekennzeichnet, daß die Gewindeöffnungen (13, 15) an dem Ende (12) des ersten Behälters (1) und in dem Austrittsstutzen (5) des zweiten Behälters (2) jeweils einen Deckel (16) aufweisen, der durch die Stange (8) durchstoßen werden kann.

4. Kartusche nach einem der Ansprüche 1 bis 3, dadurch gekennzeichnet, daß das mit dem Außengewinde versehene Ende (14) des vorderen glatten Teils (9) der Stange (8) einen Kanal (21) aufweist, der über eine den Durchtritt des einzuspritzenden Produktes gestattende Öffnung (22) seitlich an dem vorderen glatten Teil (9) der Stange ausmündet.

5. Kartusche nach einem der Ansprüche 1 bis 4, dadurch gekennzeichnet, daß die Mutter (18) einen Hebel (19) zum Drehen der Mutter aufweist.

6. Kartusche nach Anspruch 5, dadurch gekennzeichnet, daß der hintere, mit einem Außengewinde versehene Teil (10a) der Stange (8a) einen sternförmigen Querschnitt aufweist und daß der Hebel (19a) an der Mutter (18a) eine Öffnung (25) mit einem entsprechend angepaßten Querschnitt aufweist.

7. Kartusche nach einem der vorstehenden Ansprüche, dadurch gekennzeichnet, daß sie einen Kolben (107) aufweist, der verschiebbar im zweiten Behälter (101) angeordnet ist und eine axiale, mit einem Innengewinde versehene Öffnung (110) aufweist, die zur Aufnahme des mit einem Außengewinde versehenen Endes (103) der Perforierstange (102) nach dem Durchstoßen der Trennwand (104) des ersten Behälters (100) mit diesem Ende (103) dient, daß dieses mit dem Außengewinde versehene Ende (103) der Stange (102) länger ist als der mit dem Gewinde versehene Teil der axialen Gewindeöffnung (110), so daß es aus dieser Öffnung (110) vorstehen und in das Innengewinde (111a) des Austrittsstutzens (111) des zweiten Behälters (101) eingeschraubt werden kann, und daß der Kolbern eine Reihe von Öffnungen (114, 115, 116, 117) zum Durchtritt der in den Behältern enthaltenen Bestandteile in den jeweils anderen aufweist, wobei die Öffnungen so angeordnet sind, daß sie den Durchtrittsweg der Bestandteile von der Achse eines Behälters (100, 101) zur Außenseite ablenken und umgekehrt, wenn der Kolben (107) verschoben wird.

8. Kartusche nach Anspruch 7, dadurch gekennzeichnet, daß der Kolben (107) im wesentlichen kegelstumpfartig ausgebildet ist, wobei seine benachbart der Trennwand des ersten Behälters (100) liegende Basis (107a) einen Flansch (108) trägt, in dem eine ringförmige Dichtung (109) zur Abdichtung mit dem zweiten Behälter (101) eingelegt ist, daß der Kolben zwei kegelstumpfartige Elemente (112a, 112b) aufweist, die axial zueinander so angeordnet sind, daß zwischen ihnen ein Zwischenraum (113) besteht, und daß jedes Element zwei Reihen von Öffnungen (114, 115 ; 116, 117) aufweist, von denen eine Reihe nahe der Basis (107a) des Kolbens und die andere dieser gegenüberliegend angeordnet ist und die Öffnungen des einen der Elemente (112a, 112b) gegenüber den Öffnungen des anderen Elements winkelmäßig versetzt sind.

9. Kartusche nach Anspruch 8, dadurch gekennzeichnet, daß die beiden Elemente (112a, 112b) des Kolbens (107) durch Rippen (118) voneinander getrennt sind, die gekrümmte Durchtrittswege der Bestandteile begrenzen.

10. Kartusche nach Anspruch 9, dadurch gekennzeichnet, daß sich die gekrümmten Wege einerseits zwischen den verschiedenen Öffnungen (114), die auf dem äußeren Element (112a) gegenüber dessen Basis (107a) angeordnet sind, und den verschiedenen hiezu versetzten Öffnungen (117) auf dem inneren Element (112b) nahe der Basis des Kolbens und andererseits zwischen den verschiedenen Öffnungen (115), die sich am äußeren Element (112a) in der Nähe dessen Basis befinden, und den verschiedenen, hiezu versetzten Öffnungen (116) auf dem inneren Element (112b) gegenüber dessen Basis erstrecken.

11. Kartusche nach Anspruch 10, dadurch gekennzeichnet, daß die in der Nähe der Basis des Kolbens (107) angeordneten Öffnungen (115, 117) größer als die Öffnungen gegenüber dieser Basis und untereinander winkelmäßig versetzt sind.

12. Kartusche nach Anspruch 8, dadurch gekennzeichnet, daß in jeden Element (112a, 112b) die drei Öffnungen jeder Reihe gleichmäßig auf einen definierten, auf der Achse des Kolbens (107) zentrierten Kreis verteilt sind.

13. Kartusche nach einem der Ansprüche 8 bis 12, dadurch gekennzeichnet, daß die äußere Kegelstumpfförmige Oberfläche des Kolbens (107) zur inneren kegelstumpfförmigen Oberfläche (101a) des dem Austrittsstutzen (111) benachbarten Endes des zweiten Behälters (101) komplementär ist.

14. Kartusche nach einem der Ansprüche 9 bis 13, dadurch gekennzeichnet, daß die innere Oberfläche eines der Elemente des Kolbens Vorsprünge (119) zwischen den die gekrümmten Durchtrittswege der Bestandteile begrenzenden Rippen (118) aufweist.

15. Kartusche nach Anspruch 14, dadurch gekennzeichnet, daß die Rippen (118) und die Vorsprünge (119) auf der inneren Oberfläche des äußeren Elements (112a) ausgebildet sind.

16. Kartusche nach einem der Ansprüche 7 bis 15, dadurch gekennzeichnet, daß der Austrittsstutzen (111) der Kartusche mit einem Außengewinde versehen ist, mit dem er auf einen Träger aufschraubbar ist.

FIG_1

FIG_2

FIG_3

FIG_4

FIG_5

FIG_6

FIG_7

FIG.9

FIG.8

3

FIG_10

FIG_12

FIG_11

FIG_13

FIG_14

FIG_15

FIG_16

_FIG_17_

_FIG_18_

_FIG_19_

7

FIG. 20

FIG. 21

FIG. 22

FIG. 23

FIG. 24

9